# FASCICULE DE BREVET EUROPEEN

(11) **EP 2 928 386 B1**
(45) Date de publication et mention de la délivrance du brevet: **04.09.2019**
(21) Numéro de dépôt: 13815069.3
(22) Date de dépôt: 06.12.2013
(51) Int. Cl.: A61B 17/064

(54) **AGRAFE DE COMPRESSION A JAMBES CONVERGENTES**
KOMPRESSIONSKLEMME MIT ZUSAMMENLAUFENDEN SCHENKELN
COMPRESSION CLIP HAVING CONVERGENT LEGS

(30) Priorité: 06.12.2012 FR 1261733
(43) Date de publication de la demande: 14.10.2015
(73) Titulaire: In2Bones, 69130 Ecully (FR)
(72) Inventeur: COILLARD-LAVIROTTE, Jean-Yves, Paul, Albert, 69450 Saint Cyr Au Mont D'or (FR); MAUGER, Stéphane, Paul, 02600 Dommiers (FR)
(74) Mandataire: Weber, Jean-François
(86) Numéro de dépôt international: PCT/FR2013/052970
(87) Numéro de publication internationale: WO 2014/087111

(56) Documents cités:
- EP-A1- 0 867 149
- WO-A2-92/17122
- WO-A2-03/071962
- FR-A1- 2 874 166
- GB-A- 2 471 648
- US-A1- 2002 111 641

## Description

### DOMAINE TECHNIQUE

La présente invention concerne le domaine technique général des agrafes médicales susceptibles d'exercer une compression afin de réaliser la compression de deux fractions d'os ou de tissus biologiques pour assurer leur ostéosynthèse, c'est à dire leur fusion, notamment osseuse, ladite agrafe médicale comprenant au moins deux jambes s'étendant longitudinalement à partir d'un pont transversal.

La présente invention concerne une agrafe médicale de compression capable d'assurer la compression de deux fractions d'os et/ou de tissus biologiques du type tendons ou ligaments, pour assurer leur fusion comprenant au moins deux jambes s'étendant longitudinalement à partir d'un pont transversal et présentant chacune une extrémité distale, au moins une jambe occupant une position naturelle convergente formant ainsi une jambe convergente dirigée vers l'autre jambe, l'agrafe étant réalisée en un matériau présentant une élasticité suffisante pour que la jambe convergente puisse subir une déformation élastique provisoire à l'encontre de sa position naturelle convergente, par exemple pour sa mise en place, mais revenir naturellement vers ou à sa position naturelle convergente pour exercer un effort de compression, l'agrafe étant pourvue d'un moyen de déformation permettant d'exercer sur ladite au moins une jambe convergente, par l'intermédiaire d'un outil externe venant coopérer avec ledit moyen de déformation, une force antagoniste à la convergence de ladite jambe convergente pour la déformer provisoirement, le moyen de déformation étant formé par un organe de réception de l'outil externe, l'organe de réception étant disposé au niveau de la jonction entre ladite au moins une jambe convergente et le pont transversal.

### TECHNIQUE ANTERIEURE

Il est déjà connu d'avoir recours à des agrafes médicales de compression pour maintenir des fragments osseux en contact les uns avec les autres afin de réaliser progressivement leur ostéosynthèse, c'est-à-dire leur fusion osseuse par croissance cellulaire au niveau de l'interface entre les fragments osseux.

De manière classique, les fractions d'os résultants d'accidents ayant conduit à la rupture des os, sont tout d'abord remis en place par leur chirurgien puis mis en contact les uns avec les autres et maintenus en contact les uns avec les autres le long de l'interface de fracture par l'intermédiaire d'agrafes.

De manière connue, les agrafes sont généralement formées par deux jambes s'étendant longitudinalement à partir d'un pont transversal de sorte à former une agrafe en U. Après avoir réalisé deux perforations sensiblement coplanaires et parallèles dans chacune des fractions d'os dont il convient d'assurer la fusion osseuse, le chirurgien introduit chacune des jambes de l'agrafe dans les perforations jusqu'à ce que le pont transversal vienne reposer sur la surface extérieure des fractions d'os. De cette façon il est possible de maintenir en contact les deux fractions d'os à fusionner pendant un temps suffisant pour permettre la croissance cellulaire et la fusion osseuse.

Les agrafes du type mentionné précédemment donnent généralement satisfaction et permettent d'assurer à terme une fusion osseuse des fragments osseux.

Il s'avère néanmoins que les agrafes connues souffrent d'un inconvénient lié au temps nécessaire requis pour parvenir à une fusion osseuse complète. En effet, le temps requis est généralement considéré comme étant long et il est mis en relation avec la difficulté à mettre en contact les deux fragments osseux l'un avec l'autre avec une pression suffisante pour favoriser une croissance rapide des cellules osseuses. Il est en effet difficile de mettre en place correctement les agrafes classiques de l'art antérieur en U ayant des jambes parallèles et également d'obtenir une pression de contact suffisante entre les deux fragments osseux pour générer rapidement une bonne fusion osseuse.

### EXPOSE DE L'INVENTION

Les objets assignés à l'invention visent par conséquent à remédier aux différents inconvénients connus de l'art antérieur mentionnés précédemment et à proposer une nouvelle agrafe médicale de compression qui soit capable de générer facilement une compression des fractions d'os ou des tissus biologiques à fusionner l'un contre l'autre lorsque l'agrafe est insérée dans lesdites fractions d'os ou tissus biologiques.

Un autre objet de l'invention vise à proposer une nouvelle agrafe médicale de compression qui puisse être facilement mise en place par un chirurgien tout en étant capable d'exercer une compression des fractions d'os.

Un autre objet de l'invention vise à proposer une nouvelle agrafe médicale de compression qui puisse exercer une compression des fractions d'os à fusionner tout en étant particulièrement robuste et résistante à la fatigue sur le plan mécanique

Un autre objet de l'invention vise à proposer une nouvelle agrafe médicale de compression qui puisse être mise en place par un chirurgien en respectant les propriétés mécaniques originelles de l'agrafe.

Un autre objet de l'invention vise à proposer un dispositif médical formé d'un ensemble comprenant une agrafe et un organe de mise en place de l'agrafe qui permet de mettre facilement en place une agrafe médicale de compression.

Les objets assignés à l'invention sont atteints à l'aide d'une agrafe médicale de compression capable d'assurer la compression de deux fractions d'os et/ou de tissus biologiques du type tendons ou ligaments, pour assurer leur fusion comprenant au moins deux jambes s'étendant longitudinalement à partir d'un pont transversal et présentant chacune une extrémité distale, au moins une jambe occupant une position naturelle convergente formant ainsi une jambe convergente dirigée vers l'autre jambe, l'agrafe étant réalisée en un matériau présentant une élasticité suffisante pour que la jambe convergente puisse subir une déformation élastique provisoire à l'encontre de sa position naturelle convergente, par exemple pour sa mise en place, mais revenir naturellement vers ou à sa position naturelle convergente pour exercer un effort de compression, l'agrafe étant pourvue d'un moyen de déformation permettant d'exercer sur ladite au moins une jambe convergente, par l'intermédiaire d'un outil externe venant coopérer avec ledit moyen de déformation, une force antagoniste à la convergence de ladite jambe convergente pour la déformer provisoirement, le moyen de déformation étant formé par un organe de réception de l'outil externe, l'organe de réception étant disposé au niveau de la jonction entre ladite au moins une jambe convergente et le pont transversal, l'agrafe étant caractérisée en ce que l'organe de réception est formé par au moins un orifice traversant ménagé à travers l'épaisseur du pont transversal ou par au moins une découpe ouverte ménagée dans l'épaisseur du pont transversal de sorte à déboucher sensiblement à l'aplomb de ladite jambe convergente de telle manière que l'outil externe puisse être enfilé dans l'orifice ou la découpe ouverte et venir en appui le long et contre ladite jambe convergente au moins jusqu'à l'extrémité distale pour réaliser la déformation élastique provisoire, ladite au moins une jambe convergente étant pourvue d'une rainure de réception de l'outil externe dont la section est conjuguée à celle de l'outil externe, ladite rainure prolongeant l'orifice traversant ou la découpe ouverte.

### DESCRIPTIF SOMMAIRE DES DESSINS

D'autres particularités et avantages de l'invention apparaîtront et ressortiront plus en détails à la lecture de la description faite ci-après, en référence aux dessins annexés, donnés uniquement à titre d'exemple illustratif et non limitatif, dans lesquels :
- La figure 1 illustre, selon une vue générale en perspective de dessus, une première variante de réalisation d'une agrafe médicale de compression conforme à l'invention.
- La figure 2 illustre, selon une vue en coupe latérale, une agrafe de compression conforme à la variante illustrée à la figure 1.
- La figure 3 illustre, selon une vue en perspective de dessous, une agrafe médicale de compression conforme à la première variante de la figure 1.
- La figure 4 illustre, selon une vue en perspective de dessus, une agrafe médicale de compression conforme à la variante illustrée à la figure 1.
- La figure 5 illustre, selon une coupe en perspective, une variante de l'agrafe de compression conforme à l'invention.
- La figure 6 illustre, selon une vue latérale en coupe, une variante de l'agrafe de compression conforme à l'invention et illustrée à la figure 5.
- La figure 7 illustre, selon une vue en perspective, une autre variante de réalisation d'une agrafe de compression conforme à l'invention.
- La figure 8 illustre, selon une vue de dessus, la variante de réalisation d'une agrafe de compression conforme à l'invention et illustrée à la figure 7.
- La figure 9 illustre de manière schématique, un ensemble formé d'une part par une agrafe médicale de compression conforme à l'invention et correspondant à la première variante de réalisation illustrée aux figures1 à 4 et d'autre part par un outil de mise en place.
- La figure 10 illustre un ensemble conforme à l'invention formé d'une part par une agrafe médicale de compression en coupe conforme à la seconde variante de réalisation illustrée aux figures 5 et 6 et d'autre part par un outil apte à coopérer avec l'agrafe.
- La figure 11 illustre un ensemble formé d'une part par une agrafe médicale de compression conforme à l'invention et correspondant à une autre variante de réalisation illustrée aux figures 7 et 8 et d'autre part par un outil apte à coopérer avec l'agrafe pour assurer sa déformation.

### MEILLEURE MANIERE DE REALISER L'INVENTION

Telle qu'illustrée aux figures, l'invention concerne une agrafe médicale de compression 1 capable d'assurer la compression de deux fractions d'os et/ou de tissus biologiques du genre tendons ou ligaments pour assurer leur ostéosynthèse.

Ainsi, l'agrafe médicale de compression1 conforme à l'invention peut être mise en place pour assurer de manière générale la fusion osseuse de deux fractions d'os où la fusion de deux ligaments ou encore de deux tendons ou encore d'une fraction d'os avec un ligament ou d'une fraction d'os avec un tendon sans pour autant sortir du cadre de l'invention.

Telle qu'illustrée de manière générale, l'agrafe médicale de compression 1 comprend deux jambes 2, 3 présentant chacune une extrémité distale 2D, 3D et une extrémité proximale 2P, 3P, lesquelles extrémités distales sont reliées à un pont transversal 4.

Ainsi, l'agrafe médicale de compression 1 selon l'invention comprend au moins deux jambes 2, 3 s'étendant longitudinalement à partir du pont transversal 4 de manière à former de manière générale une agrafe en U à deux branches.

Selon une caractéristique importante de l'invention, l'agrafe médicale de compression 1 selon l'invention comporte au moins une jambe 2, qui occupe une position naturelle convergente formant ainsi une jambe convergente 2 dirigée vers l'autre jambe 3, l'agrafe étant réalisée en un matériau présentant une élasticité suffisante pour que la jambe convergente 2 puisse subir une déformation élastique provisoire à l'encontre de sa position naturelle convergente, par exemple pour sa mise en place, mais revenir naturellement vers ou à sa position naturelle convergente pour exercer un effort de compression grâce à son élasticité intrinsèque.

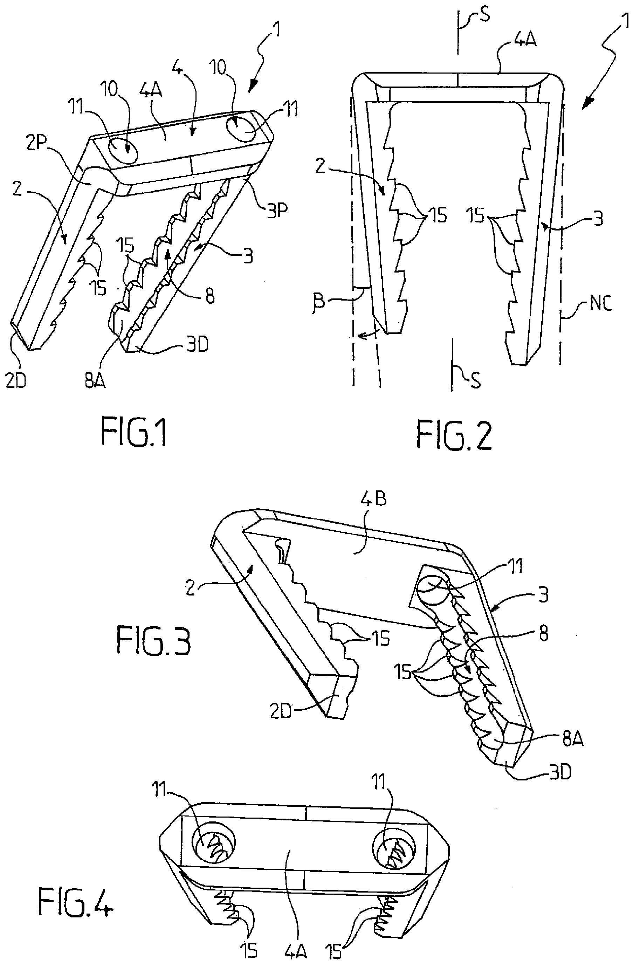

Selon la variante de réalisation illustrée aux figures 1 à 4, l'agrafe médicale de compression 1 conforme à l'invention est pourvue de deux jambes 2, 3 convergentes dirigées l'une vers l'autre ou dirigées vers le plan de symétrie principal S (figure 1) de l'agrafe. Néanmoins, l'invention concerne de manière générale une agrafe médicale de compression 1 qui peut n'être pourvue que d'une seule jambe convergente 2, l'autre jambe 3 occupant naturellement, c'est-à-dire au repos, une position non convergente de telle sorte qu'elle occupe une position NC (illustrée en ligne pointillée à la figure 2) dans laquelle la jambe 3 s'étend de manière sensiblement orthogonale au plan d'extension général du pont transversal 4 (figure 2).

Dans cette position et selon une variante non représentée aux figures, la jambe non convergente 3 forme alors au repos un angle droit avec le pont transversal 4, seule la jambe 2 formant une jambe convergente au repos.

Ainsi, sans sortir du cadre de l'invention, l'agrafe médicale de compression 1 conforme à l'invention peut comporter au moins une jambe convergente 2, ou deux jambes convergentes 2, 3.

Dans la description qui suit et dans les dessins annexés, les variantes de réalisation de l'agrafe médicale de compression 1 conforme à l'invention présentent toutes, par seul souci de simplicité, deux jambes convergentes 2, 3 sans que l'on puisse pour autant limiter l'objet de l'invention à la présence de deux jambes convergentes 2, 3.

L'agrafe médicale de compression 1 conforme à l'invention est réalisée à partir d'un matériau tel que du peek (polyétherethercétone) ou encore en un matériau métallique tel que du titane présentant une élasticité suffisante pour que la ou les jambes convergentes 2, 3 puissent être déformées ou écartées à partir de leur position de repos naturelle, en subissant une déformation élastique provisoire pour atteindre par exemple la position illustrée en ligne pointillée à la figure 2 (correspondant sensiblement à des positions dans lesquelles les jambes 2, 3 sont momentanément coplanaires et parallèles) pour reprendre ensuite naturellement la ou leur position de convergence initiale ou originelle lorsque l'effort d'écartement cesse pour exercer une compression. En outre, pour réaliser et pour faciliter la déformation de l'agrafe par le chirurgien, cette dernière est pourvue d'un moyen de déformation 10 permettant d'exercer sur ladite au moins une jambe convergente 2, 3, et par l'intermédiaire d'un outil externe 20 venant coopérer avec ledit moyen de déformation 10, une force antagoniste à la convergence de ladite jambe convergente 2 pour la déformer ou l'écarter provisoirement.

Ainsi, on dispose d'une agrafe médicale de compression 1 qui permet au chirurgien de déformer avec une grande facilité et provisoirement les agrafes conformes à l'invention rendant les jambes 2, 3 sensiblement coplanaires et parallèles, puis de mettre en place les jambes 2,3 dans des perçages préalablement ménagés dans les fragments d'os ou tissus biologiques à fusionner, pour enfin ultérieurement relâcher l'effort d'écartement pour permettre à l'agrafe de se refermer et ainsi comprimer les deux fragments osseux et/ou les tissus biologiques du genre tendons ou ligaments.

Tel qu'illustré aux figures, le moyen de déformation 10 est formé par un organe de réception de l'outil externe 20, l'organe de réception étant disposé au sein du pont transversal 4 et au niveau de la jonction entre ladite au moins une jambe convergente 2, 3 et le pont transversal 4.

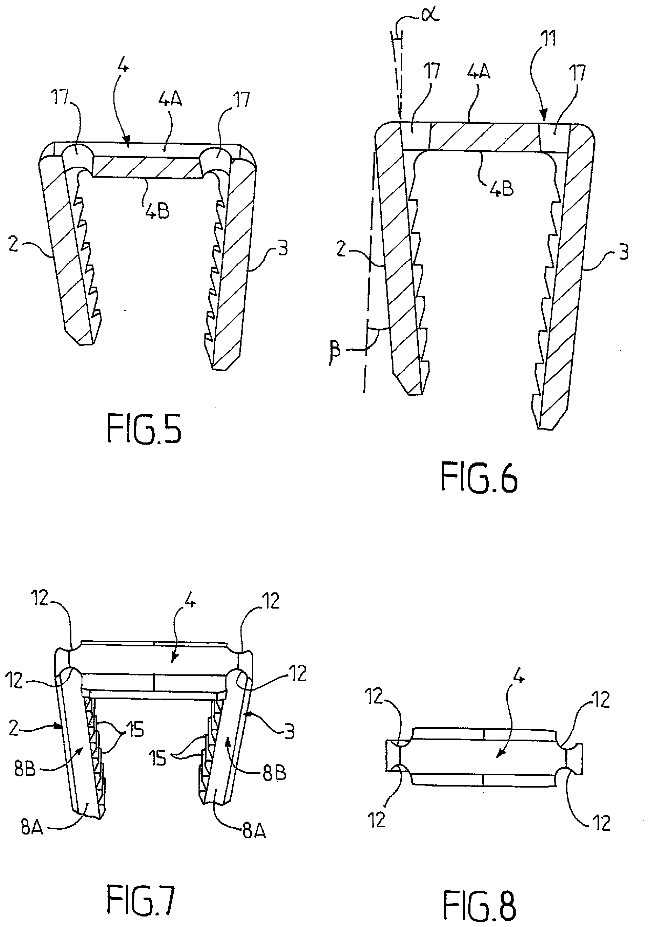

Selon l'invention, l'organe de réception est formé par au moins un orifice traversant 11 ménagé à travers l'épaisseur du pont transversal 4, tel qu'illustré à la variante des figures 1 à 4, ou par au moins une découpe ouverte 12 (figures 7, 8, 11) ménagée dans l'épaisseur du pont transversal 4 de sorte à déboucher sensiblement au droit ou à l'aplomb de ladite jambe convergente 2, 3 de telle manière que l'outil externe 20, du genre broche ou tige, puisse être enfilé dans l'orifice 11 ou la découpe ouverte et venir en appui le long et contre ladite jambe convergente 2, 3 pour réaliser la déformation élastique provisoire lorsque l'outil externe 20 est actionné.

Ainsi, l'orifice traversant 11 est ménagé à travers l'épaisseur du pont transversal 4 pour déboucher au voisinage de l'extrémité proximale 2P et/ou 3P de chaque jambe convergente 2, 3 et à la périphérie desdites jambes.

De cette manière, l'outil externe 20, tel qu'une broche 21 peut être enfilé axialement dans ledit orifice traversant 11 et venir glisser, par exemple le long de la face interne 8 de la jambe 2, 3, par exemple au moins jusqu'à l'extrémité distale 2D, 3D.

Cet agencement permet au chirurgien d'exercer un effort de déformation élastique sur chaque jambe convergente 2, 3 concernée en faisant pivoter, vers l'intérieur de l'agrafe c'est-à-dire en direction du plan de symétrie S, la broche 21 qui est en appui contre une fraction des faces internes 8 de l'orifice traversant 11 ou de la découpe ouverte 12.

Pour permettre un appui surfacique facilitant la déformation sans pour autant créer des zones de fatigue au sein de l'agrafe, chaque jambe convergente 2, 3 est pourvue, avantageusement sur sa face interne 8, d'une rainure de réception 8A de l'outil externe 20 dont la section est conjuguée à celle de l'outil externe, ladite rainure 8A prolongeant l'orifice traversant 11 ou la découpe ouverte 12.

Tel qu'illustré, et à titre de variante uniquement, la rainure 8A présente une section transversale courbe de dimension similaire à la section cylindrique de l'organe externe 20.

De manière particulièrement avantageuse et afin de renforcer la robustesse de l'agrafe et permettre une déformation élastique des jambes convergentes 2, 3 générant une fatigue minimale du matériau constitutif de l'agrafe, l'orifice traversant 11 ou les découpes ouvertes 12 sont conformés pour permettre un appui linéaire de l'outil externe 20 lors de la déformation élastique provisoire.

Selon une variante particulièrement avantageuse de l'invention illustrée aux figures 6,5 et 10 l'orifice traversant 11 a alors, une section transversale conique 17. De manière similaire, la découpe ouverte 12 a également une section transversale hémi conique.

Tel qu'illustré aux figures 2 et 6 par exemple, la section conique 17 des orifices traversant 11 ou hémi conique des découpes ouvertes 12 est disposée pour que le grand diamètre affleure la face supérieure 4A du pont transversal 4 et le petit diamètre affleure la face inférieure 4B.

La même disposition est utilisée pour la section transversale hémi conique de la découpe ouverte 12 dont le grand demi-diamètre affleure la surface supérieure 4A.

Grâce à cette disposition géométrique, l'organe externe 20 vient uniquement en appui linéaire contre une fraction du grand diamètre ou une fraction du grand demi-diamètre précédemment définit ce qui réduit fortement les efforts exercés sur la jambe convergente lors de son écartement et préserve son élasticité.

Selon une caractéristique particulièrement avantageuse de l'invention, l'angle de conicité α de la section transversale conique ou de la section transversale hémi conique est sensiblement égale à l'angle de convergence β de ladite au moins une jambe convergente 2, 3.

L'angle de convergence β et l'angle de conicité α sont par exemple compris entre 2 degrés et 20 degrés et par exemple de l'ordre de 5 degrés.

Grâce à ces choix angulaires spécifiques, il est possible d'éviter de créer des zones de contraintes au niveau des épaules de l'agrafe.

Selon une autre caractéristique avantageuse de l'agrafe selon l'invention, le pont transversal 4 est sensiblement courbe dans la situation de repos de l'agrafe de sorte à pouvoir être rectiligne lorsque les jambes 2, 3 sont sensiblement parallèles et coplanaires entre elles.

Selon cette variante de réalisation (non représentée aux figures) la courbure du pont transversal 4 présente au repos une face inférieure 4B qui est concave, la face supérieure 4A étant alors convexe.

Tel qu'illustré également aux figures, les jambes 2, 3 de l'agrafe sont avantageusement de longueur inégale, la jambe 2 étant par exemple plus courte que la jambe 3. Cette disposition favorise l'insertion des jambes 2,3 par le chirurgien. Les jambes 2, 3 de l'agrafe peuvent néanmoins être de longueur égale.

Ainsi, selon les variantes de réalisation préférentielles illustrées aux figures 1 à 11, l'agrafe selon l'invention comporte :
- au moins deux jambes convergentes 2, 3 dont la convergence est dirigée l'une vers l'autre,
- et au moins deux orifices traversant 11 ou au moins deux découpes ouvertes 12.

Selon la variante illustrée aux figures 7 à 11, l'agrafe de compression selon l'invention comporte deux paires de découpes ouvertes 12, les découpes ouvertes 12 d'une paire étant ménagées en vis à vis et de part et d'autre du pont transversal 4 pour déboucher au droit des faces latérales 8B desdites jambes convergentes 2,3.

En effet, les figures 7 et 8 illustrent une variante de réalisation dans lesquels les découpes ouvertes 12 sont ménagées aux deux extrémités opposées du pont transversal 4 et dans le prolongement des rainures 8A de telle sorte que selon cette variante de réalisation, l'agrafe médicale de compression 1 conforme à l'invention comprend quatre découpes ouvertes 12 et quatre rainures 8A qui sont associées par paire avec chaque jambe convergente 2, 3.

Il est également envisageable de réaliser une agrafe selon l'invention avec trois ou quatre jambes convergentes, voire plus, sans sortir du cadre de l'invention.

Tel qu'illustré aux figures, chaque jambe peut être pourvue d'un ensemble de dents 15 formant une double série de dents 15, par exemple situées de part et d'autre de la rainure 8A et disposées les une au-dessus des autres sur toute la longueur de la ou des jambes convergentes 2, 3.

Les dents 15 permettent d'assurer une meilleure tenue de l'agrafe, et en particulier évitent à l'agrafe de sortir du logement dans lesquels elle est insérée au sein de la masse osseuse ou des tissus biologiques.

La présente invention concerne également un ensemble formé d'une part par une agrafe médicale de compression 1 telle que décrite précédemment et d'autre part par un outil externe 20 apte à coopérer avec l'agrafe pour assurer sa déformation élastique provisoire.

Selon une version avantageuse de l'invention, l'ensemble comprenant l'agrafe médicale de compression 1 telle que décrite précédemment constitue un dispositif médical et il peut être formé par au moins un outil externe 20, du type broche ou tige 21, destiné à venir coopérer avec le moyen de déformation 10 de l'agrafe pour assurer sa déformation élastique provisoire.

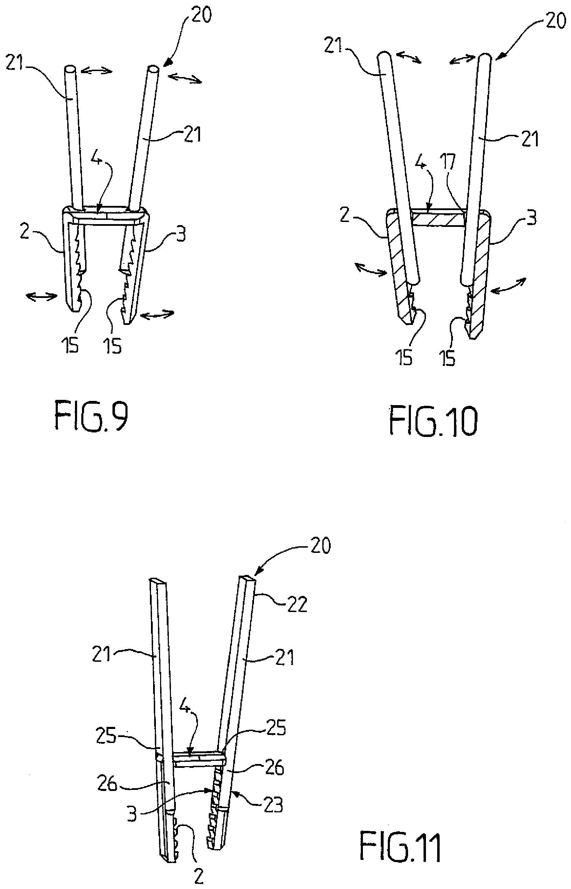

Tel qu'illustré aux figures 9, 10 et 11, l'outil externe 20 comprend au moins une broche 21, et de préférence deux broches 21 destinées à être enfilées dans les orifices traversant 11 ou dans des découpes ouvertes 12 en vue d'assurer la déformation élastique provisoire de la jambe convergente 2, 3 concernée de l'agrafe.

Les broches 21 ou tiges conformes à l'invention peuvent être réalisées en matériaux métalliques et être de section quelconque et de préférence de section circulaire, dans tous les cas d'une section appareillée à la section des rainures 8A afin de pouvoir réaliser un appui surfacique sur la ou les jambes convergentes 2, 3.

Pour la mise en place de l'agrafe selon la variante de réalisation illustrée aux figures 7 et 8, l'outil externe 20 est avantageusement pourvu d'une extrémité de préhension 22 à l'opposé de laquelle se trouve une extrémité 23 en forme de U avec une âme 25 et deux branches 26, l'âme 25 étant destinée à venir chevaucher le pont transversal 4 lorsque les branches 26 sont insérées dans les découpes ouvertes 12 en vue de réaliser la déformation élastique provisoire de la ou des jambes convergentes 2, 3 de l'agrafe concernée. Dans cette situation (Fig.11), les branches 26 sont enfilées dans les rainures 8A et viennent alors en appui surfacique au sein des rainures 8A ménagées dans les faces latérales 8B des jambes convergentes 2, 3.

De préférence, les branches 26 de l'outil externe 20 sont conçues pour être insérées dans les découpes ouvertes 12 de façon à ce que lesdites branches 26 soient noyées dans la ou les jambe(s) convergente(s) 2,3, les découpes ouvertes 12 étant prévues pour envelopper, au moins en partie, lesdites branches 26.

La présente divulgation concerne également une méthode d'implantation d'une agrafe médicale de compression conforme à l'invention selon laquelle et successivement :
- Après avoir préalablement réalisé des perforations coplanaires et parallèles, aptes à recevoir les jambes 2, 3 de l'agrafe 1, dans les fractions d'os et/ou dans les tissus biologiques du type tendons ou ligaments dont il convient d'assurer la mise en compression par l'agrafe 1,
- On déforme provisoirement la ou les jambe(s) convergente(s) 2, 3 de l'agrafe 1 à l'encontre de leur position naturelle de convergence jusqu'à ce que les jambes 2, 3 occupent une position où elles sont sensiblement parallèles et s'étendent dans le même plan,
- Tout en maintenant la ou les jambe(s) convergente(s) 2, 3 dans cette position de contrainte, on introduit alors les jambes 2, 3 dans les fractions d'os et/ou dans les tissus biologiques de type tendons ou ligaments dont on souhaite assurer la mise en compression par l'agrafe 1, en particulier dans les perforations,
- Puis on relâche les jambes (2, 3) de sorte que la ou les jambes convergente(s) (2, 3) se referme(nt) et assure(nt) la compression.

La déformation provisoire de la ou des jambes convergentes 2, 3 s'effectue donc en écartant les jambes convergentes 2, 3 jusqu'à leur faire occuper une position dans laquelle elles sont sensiblement orthogonales ou perpendiculaires au plan d'extension qui contient le pont transversal 4.

Dans cette position l'agrafe peut être facilement introduite dans les perforations réalisées préalablement par le chirurgien, l'introduction pouvant même être rendue encore plus facile si l'agrafe comporte deux jambes convergentes 2, 3 de longueurs inégales.

Dans cette situation, le chirurgien a pu en effet commencer à introduire une seule jambe dans la perforation sans avoir nécessaire à aligner immédiatement les deux jambes convergentes 2, 3 en regard de chacune des perforations.

Selon l'invention, on assure la déformation à l'aide d'un outil externe 20 qui vient prendre appui sur l'agrafe et sur les surfaces internes ou externes des jambes convergentes 2, 3 pour les écarter.

Enfin, l'outil externe 20 est enfilé dans des orifices traversant 11 ou dans des découpes ouvertes 12 ménagées dans les agrafes pour venir en appui le long de la ou des jambes convergentes 2, 3 en vue d'assurer la déformation élastique provisoire de la jambe concernée de l'agrafe.

Dès que le chirurgien relâche l'effort qu'il exerce sur les outils externes 20, l'agrafe médicale de compression 1 conforme à l'invention se referme immédiatement grâce à l'élasticité interne intrinsèque de chacune des jambes convergentes 2, 3 ce qui contribue ainsi à mettre immédiatement en compression les deux fragments osseux ou les tissus biologiques.

### POSSIBILITE D'APPLICATION INDUSTRIELLE

L'invention trouve son application industrielle dans la conception, la mise en oeuvre et la fabrication d'agrafes médicales de compression de fractions d'os et/ou de tissus biologiques.

## Revendications

1. Agrafe médicale de compression (1) capable d'assurer la compression de deux fractions d'os et/ou de tissus biologiques du type tendons ou ligaments, pour assurer leur fusion comprenant au moins deux jambes (2, 3) s'étendant longitudinalement à partir d'un pont transversal (4) et présentant chacune une extrémité distale (2D, 3D), au moins une jambe (2) occupant une position naturelle convergente formant ainsi une jambe convergente (2) dirigée vers l'autre jambe (3), l'agrafe étant réalisée en un matériau présentant une élasticité suffisante pour que la jambe convergente (2) puisse subir une déformation élastique provisoire à l'encontre de sa position naturelle convergente, par exemple pour sa mise en place, mais revenir naturellement vers ou à sa position naturelle convergente pour exercer un effort de compression, l'agrafe étant pourvue d'un moyen de déformation (10) permettant d'exercer sur ladite au moins une jambe convergente (2, 3), par l'intermédiaire d'un outil externe (20) venant coopérer avec ledit moyen de déformation (10), une force antagoniste à la convergence de ladite jambe convergente (2, 3) pour la déformer provisoirement, le moyen de déformation (10) étant formé par un organe de réception de l'outil externe (20), l'organe de réception étant disposé au niveau de la jonction entre ladite au moins une jambe convergente (2, 3) et le pont transversal (4),
l'agrafe (1) étant **caractérisée en ce que** l'organe de réception est formé par au moins un orifice traversant (11) ménagé à travers l'épaisseur du pont transversal (4) ou par au moins une découpe ouverte (12) ménagée dans l'épaisseur du pont transversal (4) de sorte à déboucher sensiblement à l'aplomb de ladite jambe convergente (2, 3) de telle manière que l'outil externe (20) puisse être enfilé dans l'orifice (11) ou la découpe ouverte (12) et venir en appui le long et contre ladite jambe convergente (2, 3) au moins jusqu'à l'extrémité distale (2D, 3D) pour réaliser la déformation élastique provisoire, ladite au moins une jambe convergente (2, 3) étant pourvue d'une rainure de réception (8A) de l'outil externe (20) dont la section est conjuguée à celle de l'outil externe (20), ladite rainure (8A) prolongeant l'orifice traversant (11) ou la découpe ouverte (12).

2. Agrafe selon la revendication 1 **caractérisée en ce que** ladite au moins une jambe convergente (2, 3) est pourvue, sur sa face interne (8) ou sur sa face latérale (8B), de ladite rainure de réception (8A) de l'outil externe (20).

3. Agrafe selon l'une quelconque des revendications 1 et 2 **caractérisée en ce que** l'orifice traversant (11) ou la découpe ouverte (12) sont conformés pour permettre un appui linéaire de l'outil externe (20) lors de la déformation élastique provisoire.

4. Agrafe selon la revendication 3 **caractérisée en ce que** l'orifice traversant (11) a une section transversale conique ou la découpe ouverte (12) a une section transversale hémi conique.

5. Agrafe selon la revendication 4 **caractérisée en ce que** l'angle de conicité α de la section transversale conique ou hémi conique est égal à l'angle de convergence β de ladite au moins une jambe convergente (2, 3).

6. Agrafe selon l'une des revendications précédentes **caractérisée en ce qu'**elle comporte :
- au moins deux jambes convergentes (2, 3) dont la convergence est dirigée l'une vers l'autre
- et au moins deux orifices traversants (11) ou au moins deux découpes ouvertes (12).

7. Agrafe selon la revendication 6 **caractérisée en ce qu'**elle comporte deux paires de découpes ouvertes (12), les découpes d'une paire étant ménagées en vis-à-vis et de part et d'autre du pont transversal (4).

8. Agrafe selon l'une des revendications précédentes **caractérisée en ce que** le pont transversal (4) est courbe dans la situation de repos de l'agrafe de sorte à pouvoir être rectiligne lorsque les jambes (2, 3) sont sensiblement parallèles entre elles.

9. Agrafe selon l'une des revendications précédentes **caractérisée en ce qu'**elle est réalisée en peek (polétherethercétone).

## Patentansprüche

1. Medizinische Kompressionsklemme (1), die zwei Knochenfrakturen und/oder Frakturen eines biologischen Gewebes des Sehnen- oder Bändertyps komprimieren kann, um ihre Fusion zu gewährleisten, umfassend mindestens zwei Schenkel (2, 3), die sich in Längsrichtung von einer Querbrücke (4) erstrecken und jeweils ein distales Ende (2D, 3D) aufweisen, wobei mindestens ein Schenkel (2) eine natürliche zusammenlaufende Stellung einnimmt, um so einen zusammenlaufenden Schenkel (2), der in Richtung des anderen Schenkels (3) ausgerichtet ist, zu bilden, wobei die Klemme aus einem Material mit ausreichender Elastizität hergestellt ist, sodass der zusammenlaufende Schenkel (2), beispielsweise für seine Einführung, eine temporäre elastische Verformung in Gegenrichtung seiner natürlichen zusammenlaufenden Stellung erfahren kann, wobei er jedoch natürlich in Richtung oder in die natürliche zusammenlaufende Stellung zurückkehrt, um eine Kompressionskraft auszuüben, wobei die Klemme mit einem Verformungsmittel (10) versehen ist, um eine Gegenkraft auf den mindestens einen zusammenlaufenden Schenkel (2, 3) durch ein äußeres Werkzeug (20), das mit dem Verformungsmittel (10) zusammenwirkt, gegen die Konvergenz des zusammenlaufenden Schenkels (2, 3) auszuüben, um ihn zeitweise zu verformen, wobei das Verformungsmittel (10) von einem Aufnahmeelement des äußeren Werkzeugs (20) gebildet wird, wobei das Aufnahmeelement an der Verbindung zwischen dem mindestens einen zusammenlaufenden Schenkel (2, 3) und der Querbrücke (4) angeordnet ist,
wobei die Klemme (1) **dadurch gekennzeichnet ist, dass** das Aufnahmeelement durch mindestens ein Durchgangsloch (11), das durch die Dicke der Querbrücke (4) angeordnet ist, oder mindestens einen offenen Einschnitt (12), der in der Dicke der Querbrücke (4) angeordnet ist, gebildet ist,
um im Wesentlichen vertikal über dem zusammenlaufenden Schenkel (2, 3) hervorzutreten, sodass das externe Werkzeug (20) in das Loch (11) oder in den offenen Einschnitt (12) eingefädelt werden kann und entlang und gegen den zusammenlaufenden Schenkel (2, 3) zumindest bis zum distalen Ende (2D, 3D) zum Ausführen der vorläufigen elastischen Verformung getragen werden kann, wobei der mindestens eine zusammenlaufende Schenkel (2, 3) mit einer Aufnahmenut (8A) des äußeren Werkzeugs (20) versehen ist, dessen Querschnitt mit demjenigen des äußeren Werkzeugs (20) konjugiert ist, wobei die Nut (8A) das Durchgangsloch (11) oder den offenen Einschnitt (12) verlängert.

2. Klemme nach Anspruch 1, **dadurch gekennzeichnet, dass** der mindestens eine zusammenlaufende Schenkel (2, 3) an seiner Innenfläche (8) oder an seiner Seitenfläche (8B) mit der Aufnahmenut (8A) des äußeren Werkzeugs (20) versehen ist

3. Klemme einem der vorhergehenden Ansprüche 1 und 2, **dadurch gekennzeichnet, dass** das Durchgangsloch (11) oder der offene Einschnitt (12) so geformt ist, um eine lineare Lagerung des äußeren Werkzeugs (20) während der temporären elastischen Verformung zu ermöglichen.

4. Klemme nach Anspruch 3, **dadurch gekennzeichnet, dass** das Durchgangsloch (11) einen konischen Querschnitt aufweist oder der offene Einschnitt (12) einen halbkonischen Querschnitt aufweist.

5. Klemme nach Anspruch 4, **dadurch gekennzeichnet, dass** der Kegelwinkel α des konischen oder halbkonischen Querschnitts dem Konvergenzwinkel β des mindestens einen zusammenlaufenden Schenkels (2, 3) entspricht.

6. Klemme nach einem der vorhergehenden Ansprüche, **dadurch gekennzeichnet, dass** sie Folgendes umfasst:
- mindestens zwei zusammenlaufende Schenkel (2, 3), deren Konvergenz aufeinander zu gerichtet ist
- und mindestens zwei Durchgangslöcher (11) oder mindestens zwei offene Einschnitte (12).

7. Klemme nach Anspruch 6, **dadurch gekennzeichnet, dass** sie zwei Paare offener Einschnitte (12) umfasst, wobei die Einschnitte eines Paars gegenüber und auf beiden Seiten der Querbrücke (4) ausgebildet sind.

8. Klemme nach einem der vorhergehenden Ansprüche, **dadurch gekennzeichnet, dass** die Querbrücke (4) in der Ruhestellung der Klemme so gebogen ist, dass sie gerade ausgerichtet sein kann, wenn die Schenkel (2, 3) im Wesentlichen parallel zueinander sind.

9. Klemme nach einem der vorhergehenden Ansprüche, **dadurch gekennzeichnet, dass** sie aus PEEK (Polyetheretherketon) hergestellt ist.

## Claims

1. A medical compression clip (1) capable of compressing together two bone fractions and/or tendon or ligament type biological tissue fractions in order to cause them to fuse together, the clip comprising at least two legs (2, 3) extending longitudinally from a transverse bridge (4) and each having a distal end (2D, 3D), at least one leg (2) occupying a naturally converging position so as to form a converging leg (2) that is directed towards the other leg (3), the clip being made out of a material presenting sufficient elasticity to enable the converging leg (2) to be subjected to temporary elastic deformation away from its naturally converging position, e.g. in order to enable it to be put into place, while enabling it to return naturally towards or to its naturally converging position in order to exert a compression force, the clip being provided with deformation means (10) enabling a force opposing the convergence of said converging leg (2, 3) to be exerted on said at least one converging leg (2, 3) by means of an external tool (20) cooperating with said deformation means (10) in order to impart temporary deformation to the converging leg, the deformation means (10) being formed by a reception member for receiving the external tool (20), the reception member being arranged at the junction between said at least one converging leg (2, 3) and the transverse bridge (4), the clip (1) being **characterized in that** the reception member is formed by at least a through orifice (11) formed through the thickness of the transverse bridge (4) or by at least one an open cutout (12) formed in the thickness of the transverse bridge (4) so as to open out substantially plumb with said converging leg (2, 3) so that the external tool (20) can be engaged through the through orifice (11) or through the open cutout (12) and bear against and along said converging leg (2, 3) at least as far as the distal end (2D, 3D) in order to impart the temporary elastic deformation, said at least one converging leg (2, 3) being provided with a groove (8A) for receiving the external tool (20), the groove being of section that is complementary to the section of the external tool (20), said groove (8A) extending the through orifice (11) or the open cutout (12).

2. A clip according to claim 1, **characterized in that** said at least one converging leg (2, 3) is provided, on its inside face (8) or on its lateral face (8B), with said groove (8A) for receiving the external tool (20).

3. A clip according to claim 1 or claim 2, **characterized in that** the through orifice (11) or the open cutout (12) is shaped so as to enable the external tool (20) to bear linearly while imparting the temporary elastic deformation.

4. A clip according to claim 3, **characterized in that** the through orifice (11) has a conic cross-section or the open cutout (12) is of semi-conic cross-section.

5. A clip according to claim 4, **characterized in that** the cone angle α of the conic or semi-conic cross-section is equal to the angle of convergence β of said at least one converging leg (2, 3).

6. A clip according to any preceding claim, **characterized in that** it includes:
- at least two converging legs (2, 3) of convergence directed towards each other; and
- at least two through orifices (11) or at two open cutouts (12).

7. A clip according to claim 6, **characterized in that** it includes two pairs of open cutouts (12), the cutouts of a given pair being arranged facing each other on either side of the transverse bridge (4).

8. A clip according to any preceding claim, **characterized in that** the transverse bridge (4) is curved when the clip is in its rest configuration so as to enable it to be rectilinear when the legs (2, 3) are substantially mutually parallel.

9. A clip according to any preceding claim, **characterized in that** it is made of polyetheretherketone (PEEK).
